# EUROPÄISCHE PATENTANMELDUNG

(11) **EP 2 962 749 A1**
(43) Veröffentlichungstag der Anmeldung: **06.01.2016**
(21) Anmeldenummer: 14175889.6
(22) Anmeldetag: 05.07.2014
(51) Int. Cl.: B01F 17/00, C12N 1/00, C11D 3/38, C02F 11/04, C12P 5/02, C12N 1/38, B01D 19/02

(54) **Verwendung von Polymerkondensaten zur Erzeugung von Biogas**

(71) Anmelder: Roeber, Oliver, 9526 Zuckenriet (CH)
(72) Erfinder: Roeber, Oliver, 9526 Zuckenriet (CH); Olschewski, Brigitte, 12557 Berlin (DE)
(74) Vertreter: Fabry, Bernd

(57) **Zusammenfassung**

Vorgeschlagen wird die Verwendung von Polymerkondensaten, die dadurch erhältlich sind, dass man Mikroorganismen mit wässrigen Alkalibasen behandelt und die so erhaltenen Zwischenprodukte anschließend mit Triglyceriden sowie gegebenenfalls mehrwertigen Carbonsäuren und/oder Polyolen mit 2 bis 12 Kohlenstoffatomen und 2 bis 6 Hydroxylgruppen umsetzt, als Hilfsstoffe bei der Erzeugung von Biogas.

## Beschreibung

### GEBIET DER ERFINDUNG

Die Erfindung befindet sich auf dem Gebiet Biogaserzeugung und betrifft die Verwendung spezieller Hilfsstoffe sowie ein Verfahren zur Erzeugung von Biogas unter Verwendung dieser Hilfsstoffe

### STAND DER TECHNIK

Mit dem starken Ansteigen der Weltbevölkerung wird der Energiebedarf bis zum Jahre 2020 um voraussichtlich 40 % steigen. Im Verlauf der nächsten 50 Jahre wird sogar mit einer Zunahme des Energieverbrauchs auf das Dreifache gerechnet. Die Verfügbarkeit fossiler Ressourcen ist begrenzt. An erster Stelle wird Erdöl bei gleich bleibendem Anteil am gesamten Energieverbrauch vermutlich nur weitere 40 Jahre verfügbar sein, die bisher bekannten und erschließbaren Erdgasreserven reichen für etwa 60 Jahre. Um das weltwirtschaftliche Wachstum nicht zu bremsen, ist ein Umstieg auf andere Energieträger zwingend.

Biogas ist durch veränderte Rahmenbedingungen sowie technische Weiterentwicklungen eine häufig diskutierte Alternative. Durch das Erneuerbare-Energien Gesetz (EEG) bekommt der Anlagenbetreiber in Deutschland und zukünftig auch in der EU Planungssicherheit, da das erhöhte Entgelt für die in das Stromnetz eingespeiste Energie auf 20 jahre fortgeschrieben ist. Gleichzeitig verbessern Investitionsprogramme kombiniert mit zinsgünstigen Darlehen die Rentabilität solcher Anlagen. Nicht wenige Landwirte erhoffen sich durch die Biogasproduktion eine zusätzliche Einnahmequelle. Umwelt- und Klimaverträglichkeit sowie gesellschaftliche Akzeptanz lassen diesen Energieträger zu einem förderungswürdigen Baustein zur Sicherung der Energieversorgung werden.

Nach konservativen Schätzungen könnten allein in Deutschland etwa 5,5 % des Erdgasverbrauchs durch Biogas gedeckt werden, was der Versorgung von 4,4 Millionen Haushalten mit Strom und 1,7 Millionen Haushalten mit Wärme entspricht. Das Potential an Strom, das mit Biogas zu erzeugen ist, entspricht demnach einer Kraftwerksleistung von etwa 2.000 MW. Nach heutigem Standard könnten dadurch zwei Kernkraftwerke ersetzt werden. Schätzungen zur Folge, könnten in den nächsten 10 Jahren zwischen 100.000 und 200.000 Biogasanlagen als Einzelhof- oder Gemeinschaftsanlagen erstellt werden.

Vor dem Hintergrund des erheblichen wirtschaftlichen Potentials darf indes nicht übersehen werden, dass die Technologie noch keineswegs optimiert ist. Schlechte Vermischung und unzureichende Verstoffwechselung der unterschiedlichsten Einsatzstoffe in den Anlagen, hohe Restwassergehalte in den Reaktionsrückständen und unerwünschte Schaumbildung sind nur einige Faktoren, die die Effizienz der Verfahren belasten.

Die Aufgabe der vorliegenden Erfindung hat somit darin bestanden, die Verfahren zur Erzeugung von Biogas, speziell von Methan, die aus dem Stand der Technik bekannt, durch Zugabe von Hilfsstoffen dahingehend zu verbessern, dass die Vermischung, Verstoffwechselung und Covergärung der Einsatzstoffe verbessert wird, weniger Restfeuchte in den Reaktionsrückständen verbleibt und Schaumbildung unterdrückt wird, um auf diese Weise die Produktionsmenge und Verfahrensgeschwindigkeit in der Biogasherstellung im Allgemeinen und in der Methanherstellung im Besonderen zu erhöhen. Dabei bestand die weitere Anforderung darin, Zusatzstoffe zu identifizieren, die diese Eigenschaften alle gleichzeitig in sich vereinigen und dabei vollständig biologisch abbaubar und umwelttechnisch völlig neutral sind.

### BESCHREIBUNG DER ERFINDUNG

Gegenstand der Erfindung ist die Verwendung von Polymerkondensaten, die dadurch erhältlich sind, dass man Mikroorganismen mit wässrigen Alkalibasen behandelt und die so erhaltenen Zwischenprodukte anschließend mit Triglyceriden sowie gegebenenfalls mehrwertigen Carbonsäuren und/oder Polyolen mit 2 bis 12 Kohlenstoffatomen und 2 bis 6 Hydroxylgruppen umsetzt, als Hilfsstoffe bei der Erzeugung von Biogas, speziell von Methan.

Besondere Ausführungsformen der Erfindung betreffen die Verwendung der Polymerkondensate
- zur Beschleunigung der Verstoffwechslung von Kohlenwasserstoffen - sowohl aus nachwachsenden als auch petrochemischen Quellen - und Proteinen;
- als Emulgatoren;
- zur Entwässerung und Reduzierung der Trockenmassenrückstände der Einsatzstoffe; speziell von Klär- und Faulschlämmen;
- als Entschäumer.

Die Polymerkondensate werden dabei üblicherweise in Mengen von etwa 0,001 bis etwa 10 Gew.-%, vorzugsweise etwa 0,01 bis etwa 8 Gew.-% insbesondere etwa 0,1 bis etwa 5 Gew.-% und besonders bevorzugt von etwa 1 bis etwa 2 Gew.-%- bezogen auf die Einsatzstoffe - eingesetzt.

Überraschenderweise wurde gefunden, dass die Polymerkondensate schon bei einer Wirkkonzentration von 0,4 g/L eine erhebliche Beschleunigung der Vergärung von mikrokristalliner Cellulose (35 %) und eine Verbesserung der Trockenmassereduzierung im Gärprozess (26 %) bewirken. Die Polymerkondensate wirken dabei gleichzeitig als Emulgatoren und Entschäumer, d.h. sie vereinigen alle gewünschten Hilfsstoffeigenschaften gleichzeitig in sich. In einer besonders bevorzugten Ausführungsform werden Emulsionen der Polymerkondensate mit natürlichen Triglyceriden hergestellt und auf den Schaum aufgesprüht, der daraufhin sofort zusammenbricht.

Dabei beruhen die Polymerkondensate komplett auf nachwachsenden Rohstoffen, sind vollständig biologisch abbaubar (WGK 1) und umwelttechnisch völlig neutral.

### POLYMERKONDENSATE

Die Polymerkondensate die die Gruppe (a) der vorliegenden Erfindung darstellen, sind aus dem Stand der Technik bekannt. Umfangreich und mit vielen Beispielen versehen werden sie in der Europäischen Patentschrift EP 0671973 B1 (OLSCHEWSKI), die bezüglich der Herstellung der Stoffe vollumfänglich über direkte Bezugnahme in die Offenbarung eingeschlossen wird.

Die Polymerkondensate der vorliegenden Erfindung werden auf der Basis von in großen Mengen zur Verfügung stehenden Mikroorganismen wie Faulschlamm, Belebtschlamm oder Hefe, insbesondere Bäckerhefe oder Bierhefe gewonnen. Anders als im Stand der Technik sonst beschrieben, dienen diese Mikroorganismen nicht als Biokatalysatoren, die an der eigentlichen chemischen Reaktion nicht teilnehmen, sondern stellen selbst das Ausgangsmaterial dar. Denn Tatsache ist, dass beispielsweise die im Sinne der Erfindung bevorzugten Hefen nichts anderes als komplexe Mischungen von im wesentlichen Proteinen und Polysacchariden darstellen, die über zahlreiche funktionelle Gruppen für nachfolgende Reaktionsschritte verfügen.

Das Molekulargewicht dieser Ausgangsstoffe liegt im Bereich oberhalb von 2 mDa, was dazu führt, dass weder eine Löslichkeit in Wasser oder Öl besteht. Im ersten Reaktionsschritt erfolgt daher ein alkalischer Aufschluss, d.h. die Mikroorganismen werden mit wässriger hochkonzentrierter Alkalilauge, beispielsweise einer 30 Gew.-%igen Natronlauge versetzt, so dass sich ein pH-Wert im Bereich von mindestens 10, vorzugsweise von 12 bis 14 einstellt, und unterhalb der Siedetemperatur, vorzugsweise bei 70 bis 80 °C etwa 2 bis 5 Stunden gerührt. Bei diesem Aufschluss werden die hochmolekularen Ketten aufgebrochen und Bruchstücke erhalten, die gegenüber den Ausgangsstoffen nur noch etwa ein Zehntel des Molekulargewichtes aufweisen. Vorzugsweise besitzen die Bruchstücke ein Molekulargewicht im Bereich von etwa 20 bis etwa 250 kDa, insbesondere etwa 50 bis etwa 220 kDa und besonders bevorzugt etwa 150 bis etwa 200 kDa. Dabei versteht es sich, dass einzelne Bruchstücke auch deutlich höhere oder niedrigere Molekulargewichte aufweisen können. Daher verstehen sich die obigen Angaben insbesondere als Mittelwerte bezogen auf etwa 90 Gew.-% der Gesamtfraktion.

Im zweiten Verfahrensschritt, der getrennt oder als "Eintopfverfahren" durchgeführt werden kann, wird den aufgeschlossenen Mikroorganismen Stoffe zugesetzt, die mit den funktionellen Gruppen in den Molekülen abreagieren können. Dabei handelt es sich um Triglyceride, mehrwertige Carbonsäuren und/oder Polyole mit 2 bis 12 Kohlenstoffatomen und 2 bis 6 Hydroxylgruppen. Die Reaktionen finden basenkatalysiert statt; d.h. in der Regel bedarf es keines weiteren Zusatzes an Alkalihydroxid, da die Lösung noch basisch genug eingestellt ist.

Bei Zusatz der Triglyceride kommt es unter alkalischen Bedingungen zu einer Verseifung. Fettsäuren werden freigesetzt, die mit Hydroxyl- oder Aminogruppen in den Protein- oder Polyzuckern Ester- oder Amidbindungen eingehen können. Zurück bleiben vor allem Partialglyceride, die selbst oberflächenaktiv sind und ausgezeichnete Emulgatoreigenschaften aufweisen. Fettsäuren, die nicht abreagieren, liegen nach der Reaktion als Seifen vor und sind damit ebenfalls oberflächenaktiv.
Geeignete Triglyceride weisen in der Regel 6 bis 22 und vorzugsweise 12 bis 18 Kohlenstoffatome auf und können gesättigt sein oder 1, 2 oder 3 Doppelbindungen aufweisen. Typische Beispiele sind pflanzliche Öle wie Palmöl, Palmkernöl, Kokosöl, Sonnenblumenöl, Sojaöl, Olivenöl, Rapsöl, Distelöl, Leinöl oder (see)tierische Ausgangsstoffe wie Fischöl oder Rindertalg. Die Ausgangsstoffe können Jodzahlen im Bereich von 1 bis 120 aufweisen. Vorzugsweise werden Mischungen von gesättigten und ungesättigten Triglyceriden eingesetzt, wie beispielsweise (gehärtetes) Palmöl und Sonnenblumenöl oder (gehärteter) Rindertalg und Rapsöl oder teilgehärtetes Kokosöl. Besonders bevorzugte Lipidkomponenten sind Kokosöl, Rapsöl, Sonnenblumenöl, Schweineschmalz und Rindertalg sowie deren Gemiscche.

Durch den Zusatz von mehrwertigen Carbonsäuren kann es über die Säurefunktionen zur Bildung von verbrückten Spezies kommen. Als mehrwertige Carbonsäuren kommen Oxalsäure, Adipinsäure, Malonsäure, Maleinsäure und insbesondere Hydroxysäuren wie Zitronen- und Weinsäure in Betracht.

Die Polyole sind in der Lage mit Säuregruppen in den polymeren Bruchstücken abzureagieren oder überschüssige freigesetzte Fettsäuren abzufangen. Sie können noch weitere funktionelle Gruppen, insbesondere Aminogruppen, enthalten bzw. selbst mit Stickstoff modifiziert sein. Typische Beispiele sind
- Glycerin;
- Alkylenglykole, wie beispielsweise Ethylenglykol, Diethylenglykol, Propylenglykol, Butylenglykol, Hexylenglykol sowie Polyethylenglykole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Das molare Verhältnis der reaktionsfähigen Gruppen in der der Summe von Biomasse und Triglycerid einerseits und mehrwertigen Carbonsäuren und Polyolen andererseits beträgt vorzugsweise 10:1 bis 1:2 und insbesondere etwa 3:1 bis etwa 1:1. Über die Auswahl der Derivatisierungsstoffe und deren Menge lässt sich der HLB-Wert der Reaktionsprodukte in weitem Maße einstellen, so dass je nach Bedarf eher lipophile Zubereitungen (HLB-Werte 6 bis 10) oder hydrophile Mischungen (HLB-Werte 11 bis 18) erhalten werden. Bezüglich der Auswahlregeln sei wieder auf die Offenbarung in der eingangs genannten Patentschrift EP 0671973 B1 verwiesen.

Die bevorzugten Polymerkondensate werden auf Basis von Mikroorganismen erhalten, die nach dem alkalischen Aufschluss mit Triglyceriden und Zitronensäure weiter umgesetzt werden. Besonders bevorzugt sind diejenigen, bei denen die Mikroorganismen wiederum Hefen, speziell Bäckerhefe darstellen.

### WEITERE ADDITIVE

Die erfindungsgemäß zu verwendenden Polymerkondensate können im Bedarfsfall mit weiteren Hilfsstoffen, speziell nichtionischen Emulgatoren und Entschäumern eingesetzt werden. Typische Beispiele umfassen:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren und Entschäumer näher erläutert:

**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. Besonders bevorzugt sind Alkoxylate, die im Mittel 1 bis 10 Mol Propylenoxid oder 1 bis 5 Mol Propylenoxid und 1 bis 10 Mol Ethylenoxid aufweisen, da diese über besonders vorteilhafte Entschäumereigenshaften verfügen. Typische Beispiele sind Laurylalkohol+2PO, Kokosfettalkohol+8EO+2PO oder Talgfettalkohol+1PO+5EO.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremonoglycerid, Ricinolsäurediglycerid, Linolsäuremonoglycerid, Linolsäurediglycerid, Linolensäure-monoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Mischether.** Typische Beispiele für geeignete Mischether sind Umsetzungsprodukte von C₈-C₁₈, vorzugsweise C₁₂-C₁₄-Fettalkoholen mit 1 bis 10 Mol Ethylenoxid und/oder 1 bis 5 Mol Propylenoxid, die mit Butylchlorid oder Benzylchlorid endgruppenverschlossen sind.

### BIOGASERZEUGUNG

Ein weiterer Gegenstand der Erfindung betrifft ein Verfahren zur Erzeugung von Biogas durch Fermentation von Biomasse, welches sich dadurch auszeichnet, dass man der Biomasse Polymerkondensate zusetzt, die durch erhältlich sind, dass man Mikroorganismen mit wässrigen Alkalibasen behandelt und die so erhaltenen Zwischenprodukte anschließend mit Triglyceriden sowie gegebenenfalls mehrwertigen Carbonsäuren und/oder Polyolen mit 2 bis 12 Kohlenstoffatomen und 2 bis 6 Hydroxylgruppen umsetzt.

Das Verfahren kann dabei ganz oder teilweise aerob oder anaerob, in offenen wie geschlossenen Anlagen durchgeführt werden, wobei die Einsatzmenge der Polymerkondensate etwa 0,001 bis etwa 10 Gew.-%, vorzugsweise etwa 0,01 bis etwa 8 Gew.-% insbesondere etwa 0,1 bis etwa 5 Gew.-% und besonders bevorzugt von etwa 1 bis etwa 2 Gew.-%-bezogen auf die Einsatzstoff- betragen kann.

Dabei schließt die Erfindung die Erkenntnis ein, dass die Polymerkondensate auch für die Entwässerung von solchen Klär- und Faulschlämmen eingesetzt werden können, die nicht zwingend als Substrate für die Biogasherstellung genutzt werden. Die Verminderung der Restfeuchte in diesen Produkten ist ein wichtiges industrielles Ziel, weil auf diese Weise die teuren und auf petrochemischen Rohstoffen beruhenden Hilfsmittel, speziell vom Polyacrylattyp, eingespart werden können.

Biogas ist ein brennbares Gas, das durch Fermentation bzw. Vergärung von Biomasse jeder Art entsteht. Das erfindungsgemäße Verfahren kann dabei in unterschiedlichsten Anlagen durchgeführt werden, beispielsweise in Biogasanlagen, Faultürmen, Fermentern oder Kläranlagen. Als Rohstoffe werden biogene Reststoffe eingesetzt, die einen hohen Anteil an Kohlenwasserstoffen und Proteinen aufweisen. Typische Beispiele sind
- vergärbare, biomassehaltige Reststoffe wie Klärschlamm, Faulschlamm, Bioabfall, Schlachtabfälle oder Speisereste sowie Fettabscheiderinhalte;
- Wirtschaftsdünger (Silagen, Gülle, Mist)
- bisher nicht genutzte Pflanzen sowie Pflanzenteile (beispielsweise Zwischenfrüchte, Pflanzenreste und dergleichen)
- gezielt angebaute Energiepflanzen und deren Folgeprodukte wie Fette und Öle.

Dabei ergeben verschiedene Ausgangsmaterialien unterschiedliche Biogaserträge und je nach ihrer Zusammensetzung ein Gas mit variablem Methangehalt, wie die folgende **Tabelle A** zeigt.

**Tabelle A**

| Biogasertrag und Methangehalt von Biomassen | | |
|---|---|---|
| **Material** | **Biogasertrag [m³]/t Frischmasse)** | **Methangehalt** |
| Maissilage | 202 | 52 % |
| Grassilage | 172 | 54 % |
| Roggen-GPS | 163 | 52 % |
| Futterrübe | 111 | 51 % |
| Bioabfall | 100 | 61 % |
| Hühnermist | 80 | 60 % |
| Zuckerrübenschnitzel | 67 | 72 % |
| Schweinemist | 60 | 60 % |
| Rindermist | 45 | 60 % |
| Getreideschlempe | 40 | 61 % |
| Schweinegülle | 28 | 65 % |
| Rindergülle | 25 | 60 % |
| Rindermist | 45 | 60 % |
| Getreideschlempe | 40 | 61 % |
| Schweinegülle | 28 | 65 % |
| Rindergülle | 25 | 60 % |

Biogas entsteht durch den natürlichen Prozess des mikrobiellen Abbaus organischer Stoffe unter anaeroben Bedingungen. Dabei setzen Mikroorganismen die enthaltenen Kohlenhydrate, Proteine und Lipide in die Hauptprodukte Methan und CO₂ um. Der Prozess besteht aus mehreren Stufen, die jeweils von Mikroorganismen verschiedener Stoffwechseltypen durchgeführt werden. Polymere Bestandteile der Biomasse, wie Cellulosen, Lignine oder Proteine, werden zunächst durch mikrobielle Exoenzyme zu monomeren Stoffen abgebaut. Niedermolekulare Stoffe hingegen werden durch gärende Mikroorganismen in Alkoholen, organische Säuren, Kohlendioxid und Wasserstoff umgewandelt. Die Alkohole und organischen Säuren werden anschließend durch acetogene Bakterien zu Essigsäure und Wasserstoff umgesetzt. In der letzten Stufe werden durch methanogene Archaeen aus Kohlendioxid, Wasserstoff und Essigsäure die Endprodukte Methan und Wasser gebildet. Der Prozess ist übersichtsweise in **Schema 1** wiedergegeben.

Die Zusammensetzung von Biogas ist sehr unterschiedlich, weil sie von der Substratzusammensetzung und der Betriebsweise des Faulbehälters abhängt. Vor der Biogasaufbereitung besteht die Gasmischung aus den Hauptkomponenten Methan und Kohlendioxid. In Spuren sind meist auch Stickstoff, Sauerstoff, Schwefelwasserstoff, Wasserstoff und Ammoniak enthalten. Wertvoll im wassergesättigt anfallenden Biogas ist das zu rund 60 % enthaltene Methan. Je höher dessen Anteil ist, desto energiereicher ist das Gas. Nicht nutzbar sind das Kohlendioxid und der Wasserdampf. Im Rohbiogas störend sind vor allem Schwefelwasserstoff und Ammoniak. Sie werden bei der Biogasaufbereitung vor der Verbrennung entfernt, um Gefährdungen des Menschen, Geruchsbelästigungen sowie Korrosion in Motoren, Turbinen und nachgeschalteten Komponenten (unter anderem Wärmetauscher) zu verhindern.

### BEISPIELE

### HERSTELLBEISPIELE

### HERSTELLBEISPIEL H1: POLYMERKONDENSAT A

400 g Brauhefe (30 Gew.-%ig) wurden mit 150 g 40 Gew.-%iger wässriger Natronlauge und 40 g PEG 300 versetzt und 30 Minuten lang bei einer Temperatur von 80 °C gerührt. Dann wurden 50 g Kokosfett (Jodzahl <1) und 75 g Kokosöl (Jodzahl 10) hinzugegeben und der Ansatz für weitere 3 Stunden bei einer Temperatur von 75 °C gerührt. Nach dem Abkühlen wurden die Reaktionsprodukte in Form einer bräunlichen wässrigen Lösung erhalten, die einen Feststoffgehalt von etwa 43 Gew.-% und einen pH-Wert von 10,5 aufwies.

### HERSTELLBEISPIEL H2: POLYMERKONDENSAT B

450 g Bäckerhefe (30 Gew.-%ig) wurden mit 180 g 40 Gew.-%iger wässriger Natronlauge und 30 Minuten lang bei einer Temperatur von 80 °C gerührt. Dann wurden 18 Gramm Zitronensäure und 9 Gramm Glycerin sowie Kokosfett (teilgehärtet) hinzugegeben. Dann wurden 190 g Schweineschmalz bzw. gehärteter Rindertalg (Jodzahl < 1; Säurezahl 4) 72 g Sonnenblumenöl und 40 g Rapsöl hinzugegeben und der Ansatz für weitere 3 Stunden bei einer Temperatur von 80 °C gerührt. Zwischenzeitlich wurden noch einmal 4,5 Gramm Natronlauge hinzugegeben. Nach dem Abkühlen wurden die Reaktionsprodukte in Form einer bräunlichen wässrigen Lösung erhalten, die einen Feststoffgehalt von etwa 51 Gew.-% und einen pH-Wert von 12,0 aufwies.

### HERSTELLBEISPIEL H3: POLYMERKONDENSAT C

400 g Bäckerhefe (25 Gew.-%ig) wurden mit 120 g 40 Gew.-%iger wässriger Natronlauge sowie mit 20 g Zitronensäure und 10 g Sorbit versetzt und 30 Minuten lang bei einer Temperatur von 80 °C gerührt. Dann wurde eine Mischung aus 100 g Milchfett und 20 g gehärtetem Palmöl (Jodzahl < 3) hinzugegeben und der Ansatz für weitere 3 Stunden bei einer Temperatur von 70 °C gerührt. Nach dem Abkühlen wurden die Reaktionsprodukte in Form einer bräunlichen wässrigen Lösung erhalten, die einen Feststoffgehalt von etwa 47 Gew.-% und einen pH-Wert von 9,5 aufwies.

### HERSTELLBEISPIEL H4: POLYMERKONDENSAT D

1000 g Belebtschlamm (25 Gew.-%ig) wurden mit 300 g 40 Gew.-%iger wässriger Natronlauge sowie 15 g Zitronensäure, 15 g Maleinsäure, 40 g PEG 600 sowie 20 g PEG 200 versetzt und 1 Stunde lang bei einer Temperatur von 80 °C gerührt. Dann wird eine Mischung aus 150 g Sonnenblumenöl (Jodzahl 130) und 100 g erucareiches Rapsöl (Jodzahl 100) hinzugegeben und der Ansatz für weitere 3 Stunden bei einer Temperatur von 70 °C gerührt. Nach dem Abkühlen wurden die Reaktionsprodukte in Form einer bräunlich-grauen wässrigen Lösung erhalten, die einen Feststoffgehalt von etwa 43 Gew.-% und einen pH-Wert von 9,5 aufwies.

### HERSTELLBEISPIEL H5: POLYMERKONDENSAT E

Analog Beispiel 6 wurden 1000 g Belebtschlamm (35 Gew.-%ig) werden mit 500 g 40 Gew.-%iger wässriger Natronlauge sowie 10 g Butanol, 10 g Butandiol, 40 g PEG 400, 10 g PEG 1000 und 50 g Maleinsäure versetzt und 1 Stunde lang bei einer Temperatur von 80 °C gerührt. Dann wurden 250 g Geflügelabfallfett hinzugegeben und der Ansatz für weitere 3 Stunden bei einer Temperatur von 75 °C gerührt. Nach dem Abkühlen wurden die Reaktionsprodukte in Form einer bräunlich-grauen wässrigen Lösung erhalten, die einen Feststoffgehalt von etwa 50 Gew.-% und einen pH-Wert von 10,5 aufwies.

### HERSTELLBEISPIEL H6: POLYMERKONDENSAT F

400 g Faulschlamm (90 Gew.-% Trockenmasse) wurden mit 600 g Wasser, 150 g 40 Gew.-%iger wässriger Natronlauge sowie mit 40 g PEG 300 versetzt und 30 Minuten lang bei einer Temperatur von 80 °C gerührt. Dann wurden 50 g Kokosfett (Jodzahl <1) und 75 g Kokosöl (Jodzahl 10) hinzugegeben und der Ansatz für weitere 3 Stunden bei einer Temperatur von 75 °C gerührt. Nach dem Abkühlen wurden die Reaktionsprodukte in Form einer bräunlich-grauen wässrigen Lösung erhalten, die einen Feststoffgehalt von etwa 55 Gew.-% und einen pH-Wert von 9,5 aufwies.

### ANWENDUNGSBEISPIELE

Die nachfolgenden Anwendungsbeispiele betreffen die Verwendung der zur Beschleunigung des Substratabbaus in Gärprozessen. Ziel war es, durch den Einsatz der Polymerkondensate in Biogasanlagen den Abbauprozess so zu beschleunigen, dass mehr Substrat pro Jahr eingesetzt werden kann, um damit eine höhere Stromausbeute zu generieren.

Zu diesem Zweck wurde die Wirksamkeit verschiedener Polymere auf den Biogasbildungsprozess bestimmt. Dazu wurden verschiedene Impfschlämme, Biogassubstrate sowie Polymere getestet und bewertet. Anschließend erfolgte eine Ermittlung der optimalen Einsatzkonzentration.

### UNTERSUCHUNGSMETHODE

Für die Ermittlung der Wirksamkeit der verschiedenen Polymere wurden Gärversuche mittels Eudiometer nach VDI 4630 "Vergärung organischer Stoffe" [VDI 2006] durchgeführt. Diese beinhalteten die Bestimmung des gebildeten Gasvolumens nach der Zeit und die Analyse des gebildeten Gases (CH₄, CO₂, O₂, H₂S). Darüber hinaus wurden die Trockenmasse und die organischen Trockenmasse [DIN 38414] sowie der pH-Wert des Ansatzes vor und nach dem Gärprozess ermittelt. Die verwendeten Impfschlämme wurden für die Versuche mit MKC aus einer 300 Liter Laborbiogasanlage und für die Versuche mit Modellfett aus dem Faulturm einer Kläranlage entnommen. Die Laborkläranlage wird mit NAWARO-Substraten, d.h. in der Regel mit Maissilage betrieben. Der Faulturm der Kläranlage Köthen wird mit Fettabscheiderresten als CO-Substrat betrieben. Somit ist dieser Impfschlamm für die Versuche mit Modellfett besser geeignet. Die Impfschlämme wurden vor dem Einsatz ausgegoren. Der Leerwert der Gasbildung (Impfschlamm ohne Substratzusatz) wurde bei jeder Versuchsreihe bestimmt. Auf eine Differenzbildung zum Leerwert wurde bei der Auswertung verzichtet, da der Focus auf der Untersuchung der Abbaugeschwindigkeit und nicht auf der Bewertung des Substrates lag. Zur Gewährleistung der statistischen Sicherheit wurden pro Ansatz immer drei unabhängige Eudiometer verwendet. Die Ermittlung der Trockenmassen erfolgte ebenfalls als Dreifachbestimmung. Die daraus berechneten Standardabweichungen wurden als Fehlerindikatoren in den einzelnen Ergebnisdiagrammen dargestellt. Die Auswertung der gewonnenen Daten wurde nach den Vorgaben der VDI 4630 vorgenommen.

### BEISPIEL 1

### Ermittlung der Wirksamkeit von Polymerkondensat A

Im Zuge der ersten Versuchsreihe sollte die Wirkung des Polymerkondensats A (in den Abbildungen auch nur als F gekennzeichnet) mit verschiedenen Ansatzkonzentrationen auf die Biogasbildungsgeschwindigkeit und die Biogaszusammensetzung bei der Vergärung von mikrokristalliner Cellulose (MKC) getestet werden. Alle relevanten Informationen zum Versuchsaufbau sind der nachfolgenden **Tabelle 1** zu entnehmen.

**Tabelle 1**

| Versuchsaufbau und Kenndaten zur Ermittlung der Wirksamkeit von A Impfschlamm | | | | | | |
|---|---|---|---|---|---|---|
| | **Impfschlamm** | **Substrat** | **Polymerkondensat** | | | |
| Bezeichnung | BMBA | MKC | A | | | |
| TS [Gew.-%] | 31,12 | | 1,87 | | | |
| oTS [Gew.-%] | 62,24 | | 77,42 | | | |
| Menge pro Ansatz* [g] | 300 | 2,0 | 0,075 | 0,150 | 0,300 | 0,900 |
| C_{WS} [mg/L] | | 9,7 10³ | 4,7 | 9,4 | 18,7 | 56,2 |

| | | | | | | |
|---|---|---|---|---|---|---|
| *) Die Ansatzmenge bezieht sich bei den Polymeren auf die Gebrauchslösung BMBA = Biomasse Biogasanlage; TS = Trockensubstanz; oTs = organische Trockensubstanz; C_{WS} = Konzentration der Wirksubstanz im Ansatz | | | | | | |

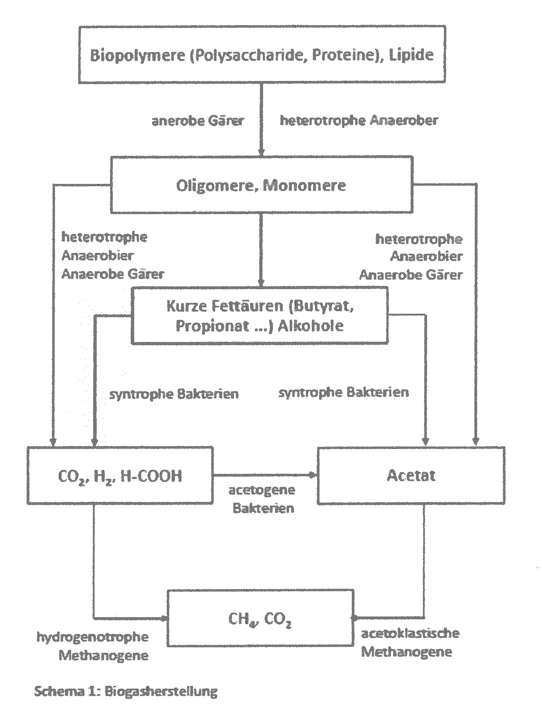

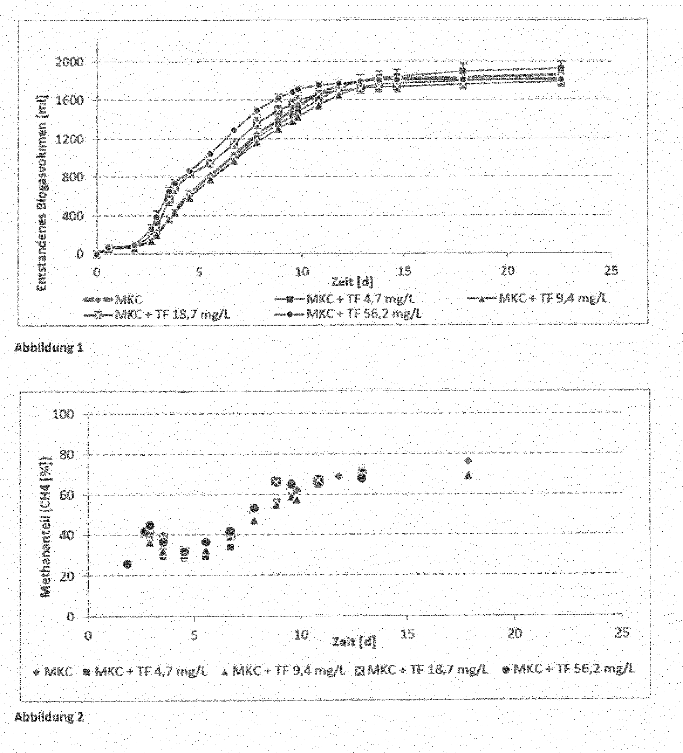

**Abbildung 1** zeigt die Wirkung von Polymerkondensat A auf das entstehende Biogasvolumen. Die Konzentrationen 4,7 mg/L und 9,4 mg/L zeigen nennenswerten keinen Effekt. Eine Beschleunigung der Gasbildung ist ab einer Konzentration von 18,7 mg/L zu verzeichnen. Die höchste eingesetzte Konzentration von 56,2 mg/L zeigte den größten Effekt. Betrachtet man die Zusammensetzung des entstandenen Gases **(****Abbildung 2****),** ist in den ersten 10 Tagen auch eine geringfügig höhere Konzentration an Methan im Biogas für die Konzentrationen 18,7 und 56,2 mg/L feststellbar.

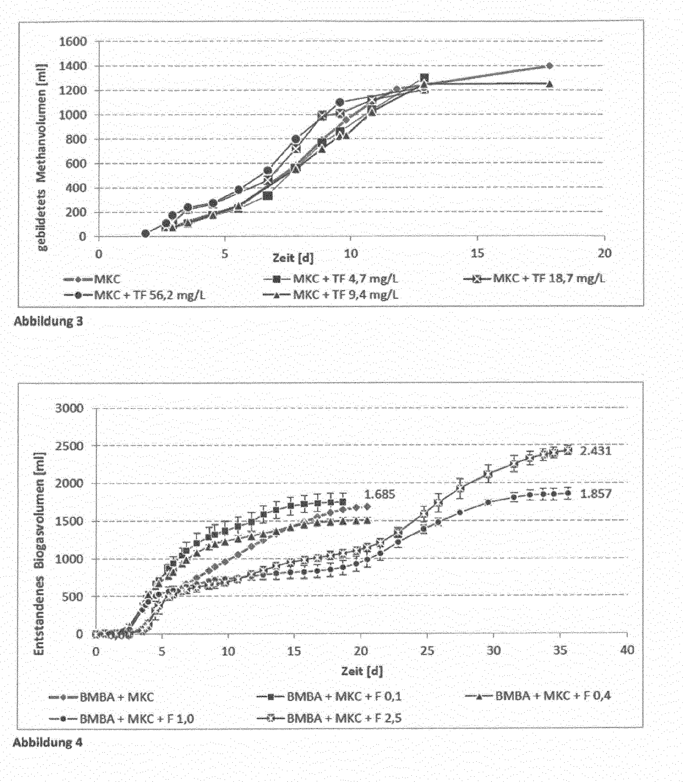

Aus dem gebildeten Biogasvolumen und dessen Methananteil, lässt sich das entstandene Methanvolumen berechnen **(****Abbildung 3****).** Diese Darstellungsweise bestätigt die bereits gewonnenen Erkenntnisse. Aufgrund dieses Versuchs wurde angenommen, dass sich durch Steigerung der Einsatzkonzentration, der beobachtete Effekt noch verstärken lässt.

### BEISPIEL 2

### Ermittlung der optimalen Wirkkonzentration von Polymerkondensat A

Aufbauend auf den Daten der Vorversuche wurde eine zweite Versuchsreihe zur Ermittlung der optimalen Einsatzkonzentration und der Inhibierungskonzentration durchgeführt. Der Konzentrationsbereich für den Einsatz von A zur Vergärung von MKC wurde hierzu angepasst. Alle relevanten Informationen zum Versuchsaufbau sind der **Tabelle 2** zu entnehmen.

**Tabelle 2**

| Versuchsaufbau und Kenndaten zur Ermittlung der Wirksamkeit von PK1 Impfschlamm | | | | | | |
|---|---|---|---|---|---|---|
| | **Impfschlamm** | **Substrat** | **Polymerkondensat** | | | |
| Bezeichnung | Faulschlamm | MKC | A | | | |
| TS [Gew.-%] | 31,12 | | 1,87 | | | |
| oTS [Gew.-%] | 62,24 | | 77,42 | | | |
| Menge pro Ansatz* [g] | 300 | 2,718 | 1,6 | 6,5 | 16,8 | 45,7 |
| C_{WS}[mg/L] | | 9,3 | 0,1 | 0,4 | 1,0 | 2,5 |

**Abbildung 4** zeigt die verschiedenen Verläufe der Biogasbildung, die aus der Zugabe unterschiedlicher Mengen des Polymerkondensats A resultieren. Die Konzentration von 0,1 g/L zeigt eine weitere Verbesserung der Biogasbildungsgeschwindigkeit im Vergleich zum Vorversuch. Bei den Konzentrationen 0,4 g/L, 1 g/L und 2,5 g/L ist eine eindeutige Inhibierung der Gärung zu verzeichnen. Bei den Ansätzen mit 1,0 g/L und 2,5 g/L ist dies deutlich zu erkennen. Das Abbruchkriterium tritt hier mit einer bis zu fünfzehntägigen Verzögerung ein. Der erneute Anstieg der Biogasbildung kann damit begründet werden, dass die zu Beginn vorliegende Inhibierungskonzentration durch Abbau des Polymers im Versuchsverlauf bis zu einer nicht inhibierenden bzw. fördernden Wirkkonzentration reduziert wird. Die Erhöhung des Maximalvolumens ergibt sich aus der Verwertung des Polymers als Substrat.

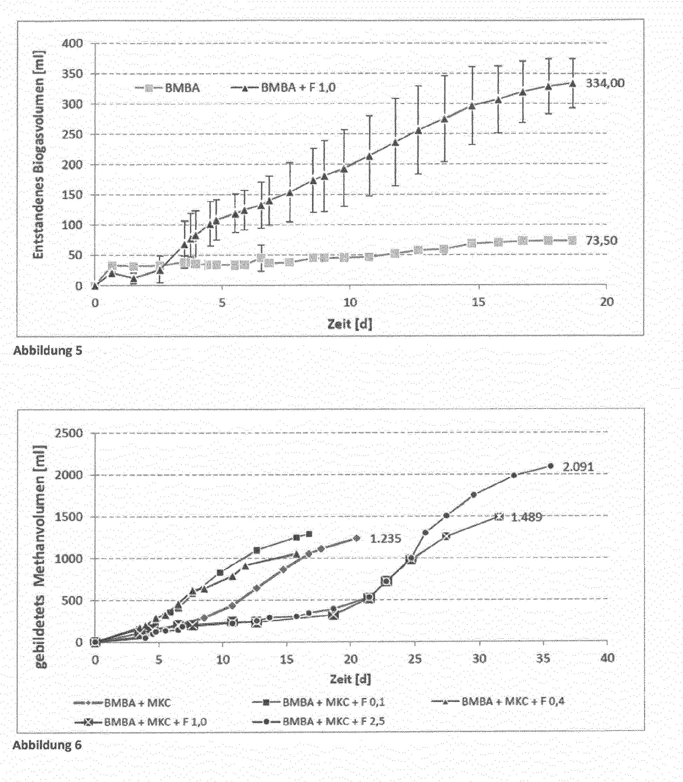

**Abbildung 5** zeigt die Biogasbildung aus 1 g/l Polymerkondensat ohne Substratzusatz. Durch den Vergleich mit einem Leerwert (300 ml Faulschlamm) kann ein Differenzvolumen von 260,5 ml berechnet werden. Bei einem Ansatzvolumen von 300 ml bedeutet das, dass aus 0,3 g Polymer 260,5 ml Biogas entstehen. Als Ergebnis erhält man 868,3 ml Biogas pro Gramm Polymer. Dies liegt im Bereich der zuvor ermittelten Werte. Der Mittelwert aus den vorherig berechneten Werten und des experimentell ermittelten Wertes beträgt 811 ml Biogas pro Gramm Polymer. Des Weiteren wird die unter Polymerzugabe eventuell effektiver verstoffwechselte Biomasse aus dem Impfschlamm bei dieser Vorgehensweise nicht berücksichtigt.

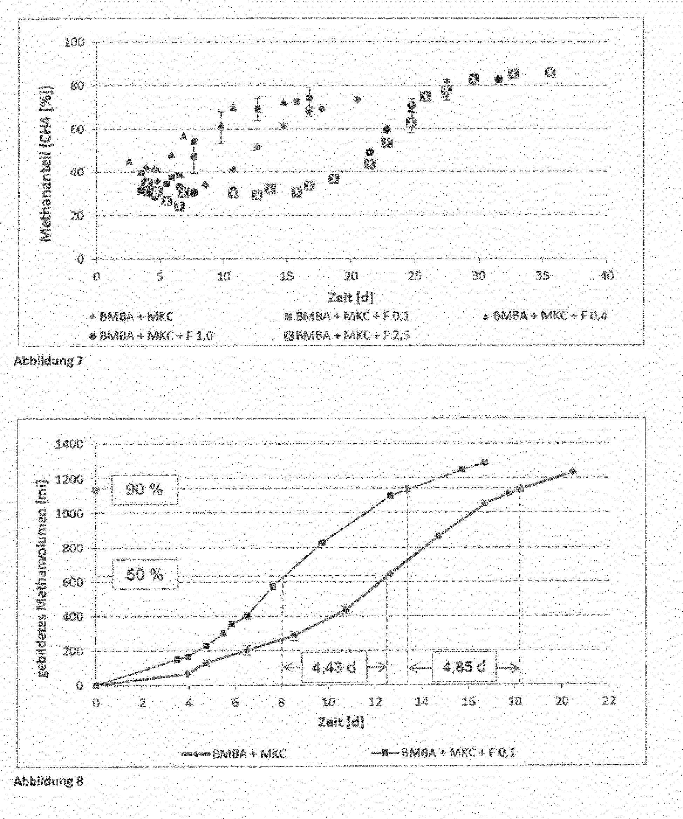

Analog zum Vorversuch wurde auch für die zweite Versuchsreihe das gebildete Methanvolumen berechnet **(****Abbildung 6****).** Auffällig ist, dass die Maximalwerte für die Konzentrationen 1,0 g/L und 2,5 g/L im Vergleich zum Ansatz ohne Polymer prozentual höher liegen. Dies ist damit zu erklären, dass durch die Vergärung des eingesetzten Polymers, Biogas mit einem hohen Gehalt an Methan entsteht (ähnlich der Vergärung von Fetten). Die Methangehalte sind in **Abbildung 7** dargestellt.

Zur besseren Veranschaulichung sind nachfolgend nur der Vergleichswert und die als optimal bestimmte Konzentration aufgeführt. Der hier auftretende Effekt ist sehr deutlich. Es kann konstatiert werden, dass durch Zugabe des Polymers die Ausgärung erheblich beschleunigt wird. Beispielhaft wurden in **Abbildung 8** und **Tabelle 3** das Erreichen von 50 % Substratabbau und 90 % Substratabbau betrachtet. Durch die Zugabe des Polymers kann die Vergärung hier um 4,43 bzw. 4,85 Tage verkürzt werden. Dies entspricht einer Beschleunigung von 35,3 % bzw. 26,58 %.

**Tabelle 3**

| Beschleunigung der Methanbildung mit Polymerkondensat A | | |
|---|---|---|
| | **Zeitdifferenz [d]** | **Zeitdifferenz [%]** |
| 30 % Ausgärung | 4,85 | 26,58 |
| 50 % Ausgärung | 4,43 | 35,30 |

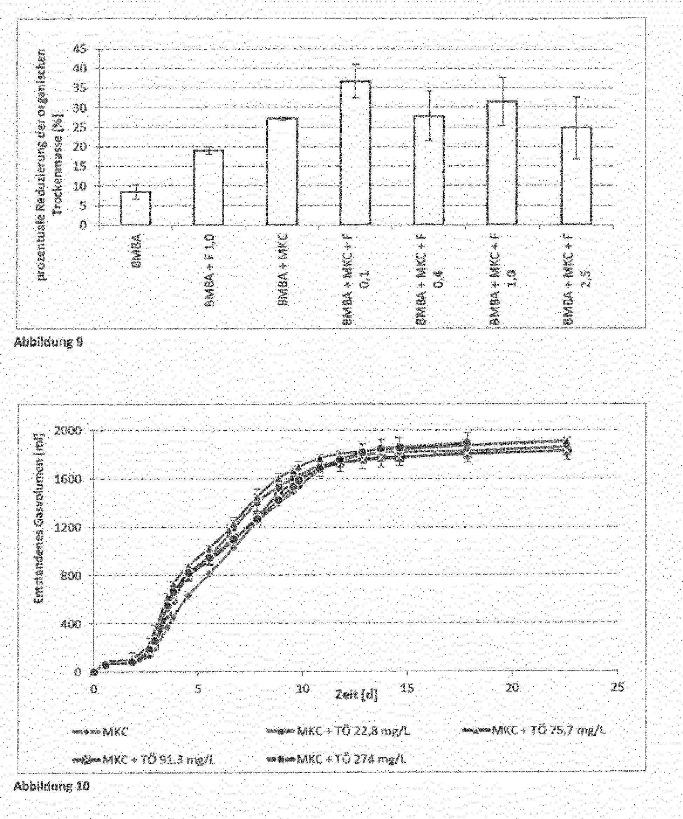

Anhand des Kurvenverlaufs und der übrigen Ergebnisse ist außerdem anzunehmen, dass das eingesetzte Polymer vor der vollständigen Verstoffwechselung des Substrates bereits abgebaut wurde. Es ist wahrscheinlich, dass eine Nachdosierung den beobachteten Effekt noch verstärken würde. Ein weiterer positiver Effekt der Polymerzugabe scheint eine bessere Verstoffwechselung des Impfschlammes selbst zu sein. Bei der Analyse der organischen Trockenmassen vor und nach der Behandlung konnte festgestellt werden, dass die bereits als optimal klassierte Konzentration von 0,1 g/L den Gärrest um 36,7 % reduziert. Dies entspricht einer Verbesserung der Reduzierung um 26 % im Vergleich zum Standardversuch ohne Polymer **(****Abbildung 9****).**

### BEISPIEL 3

### Ermittlung der Wirksamkeit von Polymerkondensat C

Analog zum ersten Vorversuch sollte auch die Wirkung des zweiten Polymerkondensats C mit verschiedenen Ansatzkonzentrationen auf die Biogasbildungsgeschwindigkeit und die Biogaszusammensetzung bei der Vergärung von mikrokristalliner Cellulose getestet werden. Alle relevanten Informationen zum Versuchsaufbau sind der nachfolgenden **Tabelle 4** zu entnehmen.

**Tabelle 4**

| Versuchsaufbau und Kenndaten zur Ermittlung der Wirksamkeit von C Impfschlamm | | | | | | |
|---|---|---|---|---|---|---|
| | **Impfschlamm** | **Substrat** | **Polymerkondensat** | | | |
| Bezeichnung | BMBA | MKC | C | | | |
| TS [Gew.-%] | 31,12 | | 9,22 | | | |
| oTS [Gew.-%] | 62,24 | | 80,81 | | | |
| Menge pro Ansatz* [g] | 300 | 2,0 | 0,075 | 0,150 | 0,300 | 0,900 |
| C_{WS}[mg/L] | | 9,7 10³ | 22,8 | 45,7 | 91,3 | 274 |

| | | | | | | |
|---|---|---|---|---|---|---|
| *) Die Ansatzmenge bezieht sich bei den Polymeren auf die Gebrauchslösung | | | | | | |

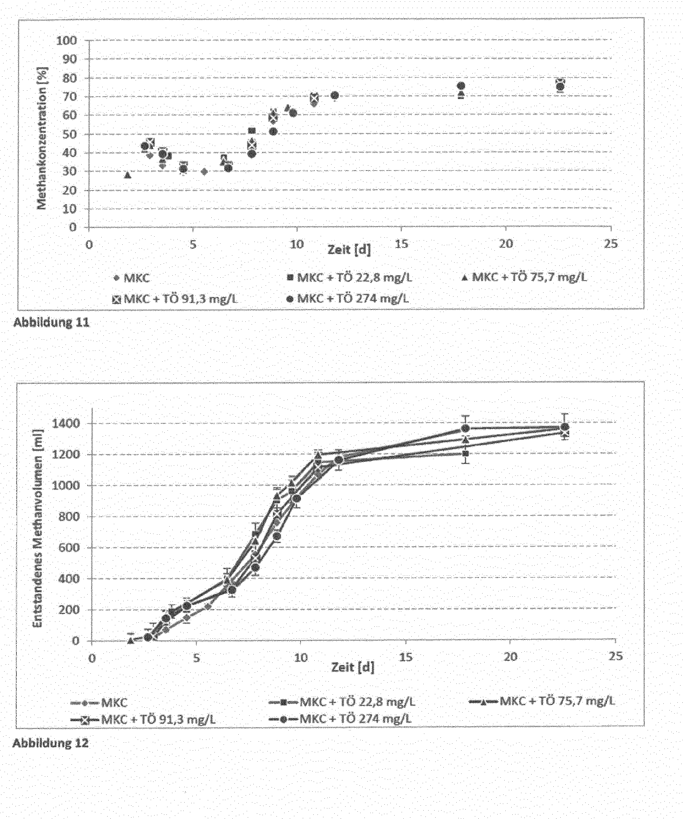

In **Abbildung 10** ist die Wirkung des Polymerkondensats B auf die Biogasbildung dargestellt. Eine geringfügige Erhöhung der Geschwindigkeit zu Beginn des Prozesses, relativiert sich bei der Berechnung des gebildeten Methanvolumens **(Abbildungen 11 und 12).**

### BEISPIEL 4

### Ermittlung der Wirksamkeit der Polymerkondensate A und C bei der Vergärung von Modellfett

Im Folgenden sollte die Wirkung der beiden Polymerkondensate A und C bei der Vergärung eines alternativen Substrates (Modellfett) getestet werden. Durch den Einsatz der Polymerkondensate sollte auch hier die Verfügbarkeit des Substrates verbessert sowie Inhomogenitäten im Reaktor vermieden werden.

Als Modellfett wurde Schweineschmalz und Frittierfett zu gleichen Teilen gemischt. Der ausgewählte Konzentrationsbereich wurde durch Versuche zur Erniedrigung des Stockpunktes ermittelt. Dazu wurde das Fettgemisch bis zur Fließfähigkeit erwärmt und mit einer definierten Menge Polymerkondensatlösung versetzt. Diese Mischung wurde bis zum Stockpunkt abgekühlt und die vorliegende Temperatur wurde notiert. Dieser Prozess wurde wiederholt, bis eine erneute Polymerzugabe den Stockpunkt wieder erhöhte. Die ermittelte Optimalkonzentration wurde dann für den Versuch verwendet. Aufbau und Kennwerte sind in **Tabelle 5** dargestellt.

**Tabelle 5**

| Versuchsaufbau und Kenndaten zur Ermittlung der Wirksamkeit von A und C Impfschlamm | | | | | |
|---|---|---|---|---|---|
| | **Impfschlamm** | **Substrat** | **Polymerkondensat** | | |
| Bezeichnung | Faulschlamm | Fett | A | | C |
| TS [Gew.-%] | 2,28 | | 1,89 | | 8,94 |
| oTS [Gew.-%] | 57,78 | | 77,4 | | 80,48 |
| Menge pro Ansatz* [g] | 300 | 2,394 | 0,48 | 5,48 | 0,12 |
| C_{WS}[mg/L] | | 7,98 | 0,03 | 0,35 | 0,035 |

| | | | | | |
|---|---|---|---|---|---|
| *) Die Ansatzmenge bezieht sich bei den Polymeren auf die Gebrauchslösung | | | | | |

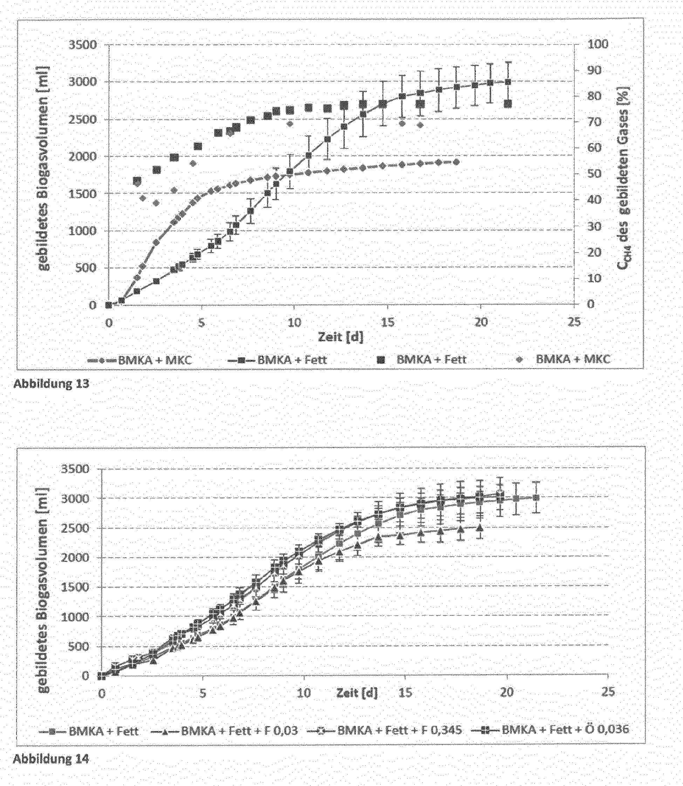

Für ein besseres Verständnis soll zunächst ein Vergleich zwischen der standardmäßig eingesetzten MKC und der verwendeten Fettlösung hergestellt werden. Wie in **Abbildung 13** dargestellt, ist der Gärverlauf beider Substrate sehr unterschiedlich. Zunächst entsteht bei der Vergärung von Fetten ein bedeutend größeres Biogasvolumen mit einem höheren Methangehalt. Der Abbau des Substrates beginnt bei der Vergärung von Fetten jedoch langsamer. Des Weiteren ist eine wesentlich höhere Abweichung zwischen den Versuchen zu beobachten. Dies ist mit der Verteilung des Substrates zu begründen. Durch das Aufschwimmen des Fettes kommt es zu Ablagerungen an der Glaswand und zu einer ungenügenden Homogenisierung, woraus sich unterschiedliche Verläufe ergeben. Durch die Mehrfachbestimmung wird dieser Fehler jedoch gut kompensiert.

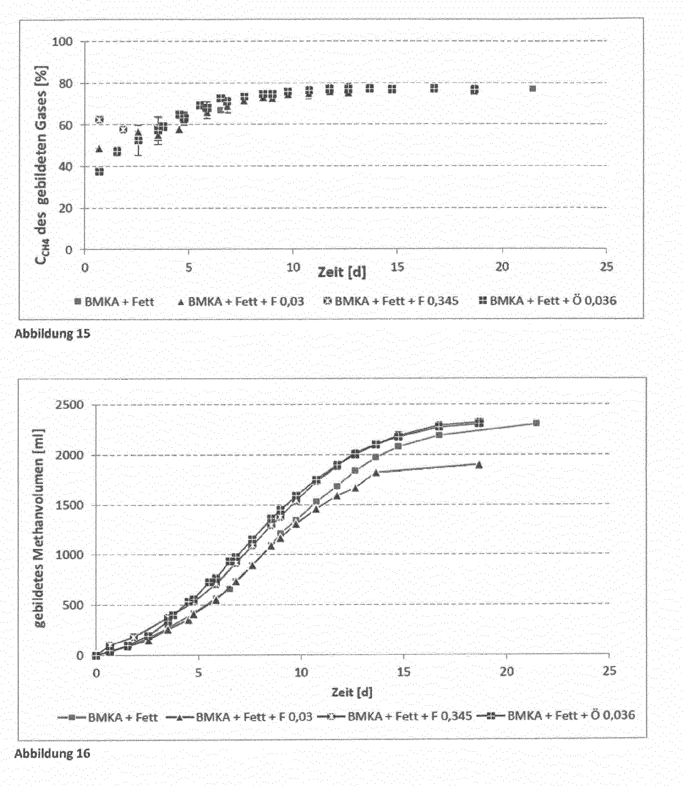

Im Folgenden sind die Ergebnisse der Versuchsreihe dargestellt. Die Kurvenverläufe liegen sehr nah beieinander und die berechneten Standardabweichungen überschneiden sich. Aus diesem Grund kann man nicht von einem deutlichen Effekt sprechen. Die Ergebnisse für die Einsatzkonzentration 0,03 g/L des Polymers 1, die einen offensichtlichen Inhibierungsverlauf beschreibt, können nicht eingeordnet werden, da dieser Effekt sich nicht bei der 10fach höheren Konzentration zeigt. Der CH₄-Gehalt des gebildeten Gases ist bei allen Ansätzen nahezu identisch **(Abbildungen 14 und 15)**

In **Abbildung 16** ist das gebildete Methanvolumen in Abhängigkeit der Zeit dargestellt. Aufgrund der geringen Unterschiede im Methangehalt ist die Beziehung der Kurvenverläufe zueinander nahezu identisch mit denen der Biogasbildung.

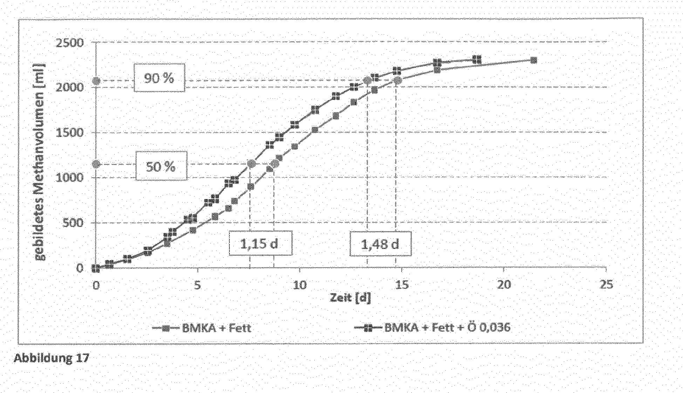

Zur Veranschaulichung ist nachfolgend nur der Vergleichswert (ohne Polymer) mit dem besten Ergebnis des Versuchs (Polymer C mit 0,036 mg/L) dargestellt. Wie bereits bei der Vergärung von MKC lässt sich hier eine Beschleunigung berechnen **(****Abbildung 17****, Tabelle 6).**

**Tabelle 6**

| Beschleunigung der Methanbildung mit Polymerkondensat B | | |
|---|---|---|
| | **Zeitdifferenz [d]** | **Zeitdifferenz [%]** |
| 30 % Ausgärung | 1,48 | 10,00 |
| 50 % Ausgärung | 1,15 | 13,07 |

### ERLÄUTERUNGEN DER ABBILDUNGEN

### Schema 1 Blogasherstellung

**Abbildung 1**
   **Wirkung von** Polymerkondensat A auf die Biogasbildung bei der Vergärung von MK; dargestellt Ist das entstandene Biogasvolumen in Abhängigkeit der Versuchsdauer. Der rote Graph stellt den Vergleichswert ohne Polymerzugabe dar.
**Abbildung 2**
   Wirkung von Polymerkandensat A auf den Methangehalt des entstandenen Biogases bei der Vergärung von MKC, dargestellt ist der Methenenteil im gebildeten Biogas in Abhängigkeit der Versuchsdauer. Der roten Punkte stellen den Methangehalt des Vergleichswertes ohne Polymerzugabe dar.
**Abbildung 3**
   Methanvolumen des entstandenen Biogases bei der Zugabe von Polymerkondensat A; dargestellt ist das entstandene Methanvolumen in Abhängigkeit der Versuchsdauer. Der rote Graph steilt den Vergleichswert ohne Polymerzugabe dar.
**Abbildung 4**
   Wirkung von Polymerkondensat A auf die Biogasbildung bei der Vergärung von MKC; dargestellt ist das entstandene Biogasvolumen In Abhängigkeit der Versuchsdauer. Der rote Graph stellt den Vergleichswert ohne Polymerzugabe dar.
**Abbildung 5**
   Bestimmung des Biogasbildungspotentials von Polymerkondensat A; dargestellt ist das entstandene Biogasvolumen In Abhängigkeit der Versuchsdauer. Der schwarze Graph stellt die Blogasbildung bei der Vergärung von 1 g/L A ohne den Zusatz von weiteren Substraten dar. Der graue Graph stellt den Vergleichswert ohne Polymer- und Substratzugabe dar.
**Abbildung 6**
   Methanvolumen des entstandenen Biogases bei der Zugabe von Polymerkondensat A (b); dargestellt ist das entstandene Methanvolumen In Abhängigkeit der Versuchsdauer. Der rote Graph stellt den Vergleichswert ohne Polymerzugabe dar.
**Abbildung 7**
   Einfluss von Polymerkondensat A auf den Methangehalt des entstandenen Biogases bei der Vergärung von MKC (b); dargestellt ist der Methananteil im gebildeten Biogas in Abhängigkeit der Versuchsdauer. Der roten Punkte stellen den Methangehalt des Vergleichswertes ohne Polymerzugabe dar.
**Abbildung 8**
   Vergleich der Methanvolumen des entstandenen Biogases zwischen der optimalen Einsatzkonzentration an Polymerkondensat A und der Standardvergärung von MKC; dargestellt ist das entstandene Methanvolumen in Abhängigkeit der Versuchsdauer. Der rote Graph stellt den Vergleichswert ohne Polymerzugabe dar.
**Abbildung 9**
   Einfluss von Polymerkundensat A auf die prozentuale Reduzierung der organischen Trockenmasse bei der Vergärung von MKC; dargestellt ist die prozentuale Differenz der organischen Trockenmasse vor und nach der Vergärung. Als Startwert wurde die organische Trockenmasse des impfschlammes vor der Substratzugabe verwendet. Somit wird die alleinige Reduzierung der Impfschlammmenge erfasst.
**Abbildung 10**
   Wirkung von Polymerkondensat C auf die Blogasbildung bei der Vergärung von MKC; dargestellt ist das entstandene Biogasvolumen In Abhängigkeit der Versuchsdauer. Der rote Graph stellt den Vergleichswert ohne Polymerzugabe dar.
**Abbildung 11**
   Wirkung von Polymerkondensat C auf den Methangehalt des entstandenen Biogases bei der Vergärung von MKC; dargestellt ist der Methananteil im gebildeten Biogas in Abhängigkeit der Versuchsdauer. Die roten Punkte stellen den Methangehalt des Vergleichswertes ohne Polymerzugabe dar.
**Abbildung 12**
   Methanvolumen des entstandenen Biogases bei der Zugabe von Polymerkondensat C; dargestellt ist das entstandene Methanvolumen In Abhängigkeit der Versuchsdeuer. Der rote Graph stellt den Vergleichswert ohne Polymerzugabe dar.
**Abbildung 13**
   Vergleich zwischen der Vergärung von Fett und MKC; dargestellt ist das entstandene Biogasvolumen In Abhängigkeit der Versuchsdauer. Die rot gekennzeichneten Werte stellen den Abbau von MKC dar.
**Abbildung 14**
   Effekt auf die Biogasbildung bei der Vergärung von Modellfett unter Polymerzugabe; dargestellt ist das entstandene Biogasvolumen In Abhängigkeit der Versuchsdauer. Der rote Graph stellt den Vergleichswert ohne Polymerzugabe dar.
**Abbildung 15**
   Effekt auf den Methangehalt bei der Vergärung von Modellfett unter Polymerzugabe; dargestellt ist der Methananteil im gebildeten Biogas in Abhängigkeit der Versuchsdauer. Der roten Punkte stellen den Methangehalt des Vergleichswertes ohne Polymerzugabe dar.
**Abbildung 16**
   Effekt auf die Methanbildung bei der Vergärung von Modellfett unter Polymerzugabe; dargestellt Ist das entstandene Methanvolumen In Abhängigkeit der Versuchsdauer. Der rote Graph stellt den Vergleichswert ohne Polymerzugabe dar.
**Abbildung 17**
   Vergleich der Methanvolumen des entstandenen Biogases zwischen der optimalen Einsatzkonzentration an Polymer 2 und der Standardvergärung von Modellfett; dargestellt ist das entstandene Methanvolumen In Abhängigkeit der Versuchsdauer. Der rote Graph stellt den Vergleichswert ohne Polymerzugabe dar.

## Patentansprüche

1. Verwendung von Polymerkondensaten, dadurch erhältlich, dass man Mikroorganismen mit wässrigen Alkalibasen behandelt und die so erhaltenen Zwischenprodukte anschließend mit Triglyceriden sowie gegebenenfalls mehrwertigen Carbonsäuren und/oder Polyolen mit 2 bis 12 Kohlenstoffatomen und 2 bis 6 Hydroxylgruppen umsetzt, als Hilfsstoffe bei der Erzeugung von Biogas.

2. Verwendung nach Anspruch 1, **dadurch gekennzeichnet, dass** man die Polymerkondensate zur Beschleunigung der Verstoffwechslung von Kohlenwasserstoffen und Proteinen einsetzt.

3. Verwendung nach Anspruch 1, **dadurch gekennzeichnet, dass** man die Polymerkondensate als Emulgatoren einsetzt.

4. Verwendung nach Anspruch 1, **dadurch gekennzeichnet, dass** man die Polymerkondensate als Hilfsstoffe zur Entwässerung und Reduzierung der Trockenmassenrückstände der Einsatzstoffe, speziell von Klär- und Faulschlämmen einsetzt.

5. Verwendung nach Anspruch 1, **dadurch gekennzeichnet, dass** man die Polymerkondensate als Entschäumer einsetzt.

6. Verwendung nach mindestens einem der Ansprüche 1 bis 5, **dadurch gekennzeichnet, dass** man Polymerkondensate einsetzt, die als Lipidkomponenten Kokosöl, Rapsöl, Sonnenblumenöl, Schweineschmalz und/oder Rindertalg enthalten.

7. Verwendung nach mindestens einem der Ansprüche 1 bis 6, **dadurch gekennzeichnet, dass** man sie in Mengen von 0,001 bis 10 Gew.-% - bezogen auf die Einsatzstoffe - einsetzt.

8. Verfahren zur Erzeugung von Biogas durch Fermentation von Biomasse, **dadurch gekennzeichnet, dass** man der Biomasse Polymerkondensate zusetzt, die durch erhältlich sind, dass man Mikroorganismen mit wässrigen Alkalibasen behandelt und die so erhaltenen Zwischenprodukte anschließend mit Triglyceriden sowie gegebenenfalls mehrwertigen Carbonsäuren und/oder Polyolen mit 2 bis 12 Kohlenstoffatomen und 2 bis 6 Hydroxylgruppen umsetzt.

9. Verfahren nach Anspruch 8, **dadurch gekennzeichnet, dass** man die Erzeugung des Biogases in Biogasanlagen, Faultürmen, Fermentern oder Kläranlagen durchführt.

10. Verfahren nach den Ansprüchen 8 und/oder9, **dadurch gekennzeichnet, dass** man als Biomasse biogene Reststoffe einsetzt, die einen hohen Anteil an Kohlenwasserstoffen und Proteinen aufweisen.

11. Verfahren nach Anspruch 10, **dadurch gekennzeichnet, dass** man als biogene Reststoffe Klärschlamm, Bioabfall, Schlachtabfälle, Speisereste, Silagen, Gülle, Mist, Zwischenfrüchte, Pflanzenreste, Energiepflanzen sowie Fette und Öle einsetzt.

12. Verfahren nach mindestens einem der Ansprüche 8 bis 11, **dadurch gekennzeichnet, dass** man es anaerob durchführt.

13. Verfahren nach mindestens einem der Ansprüche 8 bis 11, **dadurch gekennzeichnet, dass** man es aerob durchführt.

14. Verfahren nach mindestens einem der Ansprüche 8 bis 13, **dadurch gekennzeichnet, dass** man Polymerkondensate einsetzt, die die als Lipidkomponenten Kokosöl, Rapsöl, Sonnenblumenöl, Schweineschmalz und/oder Rindertalg enthalten.

15. Verfahren nach mindestens einem der Ansprüche 8 bis 14, **dadurch gekennzeichnet, dass** man die Polymerkondensate in Mengen von 0,001 bis 10 Gew.-% - bezogen auf die Einsatzstoffe - einsetzt.
